# EUROPEAN PATENT SPECIFICATION

(11) **EP 1 333 828 B1**
(45) Date of publication and mention of the grant of the patent: **02.07.2008**
(21) Application number: 01996370.1
(22) Date of filing: 13.11.2001
(51) Int. Cl.: A61K 31/4174, A61K 31/4164, A61K 31/4178, A61P 25/14

(54) **PREVENTION OF DEVELOPMENT OF DYSKINESIAS**
VORBEUGUNG DER ENTWICKLUNG VON DYSKINESIE
PREVENTION DU DEVELOPPEMENT DES DYSKINESIES

(30) Priority: 14.11.2000 US 248004 P
(43) Date of publication of application: 13.08.2003
(73) Proprietor: ORION CORPORATION, 02200 Espoo (FI)
(72) Inventor: HAAPALINNA, Antti, FIN-20360 Turku (FI); JUHILA, Juuso, FIN-20540 Turku (FI); SIRVIÖ, Jouni, FIN-70820 Kuopio (FI)
(74) Representative: Sexton, Jane Helen
(86) International application number: PCT/FI2001/000989
(87) International publication number: WO 2002/039991

(56) References cited:
- BRIAN HENRY PHD ET AL: "The alpha 2-adrenergic receptor antagonist idazoxan reduces dyskinesia and enhances anti-parkinsonian actions of L-Dopa in the MPTP-lesioned primate model of parkinson's disease" MOVEMENT DISORDERS, vol. 14, no. 5, 1999, pages 744-753, XP002902469
- GRONDIN R ET AL : "Noradrenoceptor antagonism with idazoxan improves L-dopa-induced dyskinesias in MPTP monkeys." NAUNYN-SCHMIEDEBERG'S ARCH PHARMACOL, vol. 361, 2000, pages 181-186, XP002902470
- DATABASE WPI Section Ch, Week 199838 Derwent Publications Ltd., London, GB; Class B02, AN 1998-439852 XP002902471 & FR 2 759 291 A (FABRE MEDICAMENT SA) 14 August 1998 (1998-08-14)
- DATABASE STN INTERNATIONAL [Online] file medline; RASCOL O ET AL : "Idazoxan, an alpha 2-antagonist and L-dopa-induced dyskinesias in patients with parkinson's disease." retrieved from MEDLINE, accession no. 2001433881 Database accession no. 21374324 XP002902472 & MOVEMENT DISORDERS , vol. 16, no. 4, July 2001 (2001-07), pages 708-713,
- DATABASE STN INTERNATIONAL [Online] file medline; FOX S H ET AL: "Neutral mechanisms underlying peak-dose dyskinesia induced by levodopa and apomorphine are distinct: Evidence from the effects of the alpha 2-adrenoceptor antagonist idazoxan." retrieved from MEDLINE, accession no. 2001433872 Database accession no. 21374315 XP002902473 & MOVEMENT DISORDERS, vol. 16, no. 4, July 2001 (2001-07), pages 642-650,
- DATABASE BIOSIS [Online] BIOSCIENCES INFORMATION SERVICE, PHILADELPHIA, PA, US; 2001 SAVOLA J M ET AL: "JP-1730, a novel alpha2-adrenergic antagonist, reduces L-DOPA-induced dyskinesia in animal models of Parkinson's disease." Database accession no. PREV200100499788 XP002902474 & SOCIETY FOR NEUROSCIENCE ABSTRACTS, vol. 27, no. 1, 2001, page 531 31st Annual Meeting of the Society for Neuroscience;San Diego, California, USA; November 10-15, 2001 ISSN: 0190-5295

## Description

The present invention relates to a method for preventing the development of sensitization caused by chronic use of dopaminergic agents. Especially, the present invention relates to the use of alfa2-adrenoceptor antagonists in the prevention of the development of sensitization caused by chronic use of dopaminergic agents.

Additional objects and advantages of the invention will be set forth in part in the description which follows, and in part will be obvious from the description, or may be learned by practice of the invention.

### BACKGROUND OF THE INVENTION

Dopamine is a neurotransmitter that influences on many behavioural functions such as locomotor activity and learning and it is involved in neuropsychiatric disorders such as Parkinson's Disease and schizophrenia (Beninger 1983). Stimulants like amphetamine and cocaine enhance dopamine release in the CNS by inhibition of dopamine uptake from the synaptic cleft. When amphetamine is administered repeatedly in daily doses, the increase in motor activity is higher than after one single dose, a phenomenon that is called amphetamine sensitization. This phenomen is connected with the development of drug dependency, but it may also be considered as a dyskinesia caused by chronic use of dopaminergic agents.

In animal models of α₂-adrenoceptor antagonists, such as idazoxan and atipamezole, are known to have therapeutic effects on the symptoms of Parkinson's Disease (PD). In animal models of PD, they also after acute administration potent the motor responses of dopaminergic agents such as, apomorfine, L-3,4dihydroxyphenylalanine(L-dopa) and amphetamine. In addition, in PD patients and animal models where the dyskinesias are developed after chronic administration of L-dopa, α₂-adrenoceptor antagonists have decreased the dyskinesias by enhancing inhibition in so called indirect pathway of basal ganglia which is influenced by D2 dopamine receptors (Brotchie, J.M., Parkinson's Disease Advances in Neurology, Vol. 80., in Advances in Understanding the Neural Mechanisms Underlying L-Dopa-Induced Dyskinesias, Edited by Gerald M. Stem, Lippincott William & Wilkins, Philadelphia 1999). However, the most effective way to control dyskinesias in patients is to prevent their development during dopaminergic treatment. The development of dyskinesia has been proposed to involve the overactivity of so called direct pathway of basal ganglia which is influenced by D 1 dopamine receptors. According to the knowledge of the inventors the use of alpha2-adrenoceptor antagonist in the prevention of the development of dyskinesias has not been suggested or shown before.

### BRIEF DESCRIPTION OF THE DRAWINGS

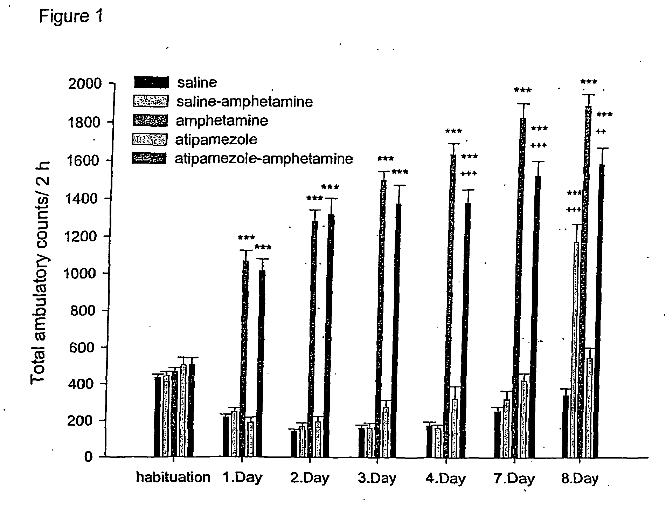

Figure 1 shows the mean 2-h overall ambulatory activity counts ± S.E.M. after six repeated administrations of D-amphetamine 2 mg/kg s.c. and the effect of atipamezole 1 mg/kg s.c. pre-treatment 20 minutes before D-amphetamine challenge, n= 20-72. Groups: saline (days 1-8 saline); saline-amphetamine (days 1-7 saline and day 8 D-amphetamine); amphetamine (days 1-8 D-amphetamine); atipamezole (days 1-8 atipamezole before saline); atipamezole-amphetamine (days 1-8 atipamezole before D-amphetamine). Statistical significances: locomotor activity of the group compared to saline-saline -group (***P<0.001, **P<0.01 and *P<0.05) and locomotor activity of the group compared to amphetamine-amphetamine -group (⁺⁺⁺P<0.001, ⁺⁺P<0.01 and ⁺P<0.05).

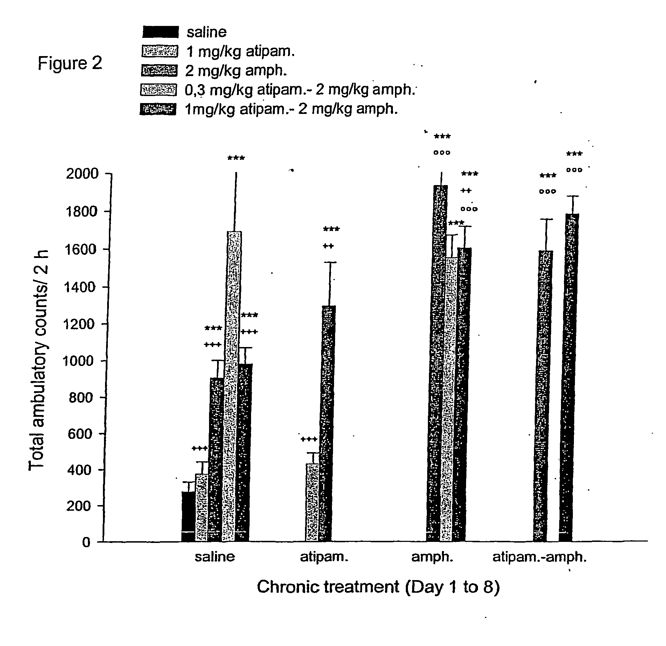

Figure 2 shows the mean 2-h overall ambulatory activity counts ± S.E.M. at day 9, n= 5-29. Chronic treatment groups: saline (days 1-8 saline); atipam. (days 1-8 atipamezole 1 mg/kg); amph. (days 1-8 D-amphetamine 2 mg/kg); atipam,- amph. (days 1-8 atipamezole 1 mg/kg 20 minutes before D-amphetamine 2 mg/kg). All drugs were administrated subcutaneously in volume 0,1 ml. Drug treatments at day 9: saline (saline 20 min before saline); 1 mg/kg atipam. (atipamezole 1 mg/kg 20 min before saline); 2 mg/kg amph. (saline 20 min before D-.amphetamine 2 mg/kg); 0,3 mg/kg atipam.- 2 mg/kg amph. (atipamezole 0,3 mg/kg 20 min before D-amphetamine 2 mg/kg); 1 mg/kg atipam.- 2 mg/kg amph. (atipamezole 1 mg/kg 20 min before D-amphetamine 2 mg/kg); Statistical significances: locomotor activity of the group compared to saline-saline -group (***P<0.001, **P<0.01 and *P<0.05), locomotor activity of the group compared to amph.-2 mg/kg amph. -group (⁺⁺⁺P<0.001, ⁺⁺P<0.01 and ⁺P<0.05) and locomotor activity of the group compared to the chronic saline group with same drug treatment at day 9 (°°°P<0.001, °°P<0.01 and °P<0.05).

### DETAILED DESCRIPTION OF THE INVENTION

Applicants have surprisingly discovered that an alfa2-adrenoceptor antagonist, atipamezole, reduced the development and expression of sensitization (motor overactivity) when given chronically in combination with a dopaminergic stimulator, D-amphetamine, in mice. Thus, alfa2-adrenoceptor antagonists such as atipamezole, and their pharmacologically acceptable esters or salts, can be used for prevention of development of sensitizational conditions caused by choric use of dopaminergic agents. The sensitizational conditions include e.g., dyskinesias and psychosis developed by chronic use of dopaminergic agents such as, apomorfine, amphetamine, and L-dopa.

Nigrostriatal dopaminergic neurons from substantia nigra to the dorsal striatum are believed to be central in the modulation of extrapyramidal motor processes. This circuitry is disturbed in PD and cause symptoms typical to PD like tremor, rigidity and difficulties in the initiation of motor actions. L-dopa has been used to relieve symptoms of PD. However, many complications are observed after continuous treatment with L-dopa, of which the most common are abnormal involuntary movements called dyskinesia (Barbeau 1974). The plastic changes in dopaminergic system controlling motor responses are thought to be responsible for development of dyskinesia. Alfa2-adrenoceptor antagonists, such as atipamezole are found to enhance neuronal plasticity (Puurunen K, Jolkkonen J, Sirviö J, Haapalinna A, Sivenius J. An alpha-2 adrenergic antagonist, atipamezole, facilitates behavoral recovery after focal cerebral ischemia in rats. Neuropharmacology 40: 597-606, 2001). Furthermore, the activation of D1 dopamine receptors and the blockade of alpha-2 adrenoceptors can cause the same kind of effect in the second messanger systems of basal ganglia. Thus, repeated administration of alfa2-adrenoceptor antagonist might be inactive or even enhance the development of dyskinesias. Locomotor hyperactivity caused by chronic activation of dopaminergic transmission by amphetamine is also a dysfunction in motor activity and is also due to sensitization effect like dyskinesia seen after chronic L-dopa treatment. The present invention provides a new solution in the pharmacotherapy of Parkinson's disease with alfa2-adrenoceptor antagonist by preventing the development of dyskinesia caused by chronic use of dopaminergic agents.

Alfa2-adrenoceptor antagonist of the invention include, without limitation, atipamezole, idazoxan, efaroxan and their analogs and pharmaceutically acceptable salts. 4-(2-ethyl-2,3-dihydro-1H-inden-2-yl)-1H-imidazole, known as atipamezole, and its pharmaceutically acceptable acid addition salts with inorganic and organic acids generally used for the purpose, are described in U.S. Patent. No. 4,689,339. The halogenated analogs of atipamezole, for example 4-(2-ethyl-5-fluoro-2,3-dihydro-1H-inden-2-yl)-1H-imidazole and 4-(2-ethyl-5,6-difluoro-2,3-dihydro-1H-inden-2-yl)-1H-imidazole and their pharmaceutically acceptable acid addition salts have been discribed in U.S. Patent No. 5,498,623. Idazoxan, 2-(2-(1,4-benzodioxanyl))-2-imidazoline, and efaroxan, 2-(2-ethyl-2,3-dihydro-2-benzofuranyl)-4,5-dihydro-1H-imidazole and their pharmaceutically acceptable acid addition salts, are described in U.S. Patents Nos. 4,818,764 and 4,411,908, respectively.

To achieve optimal results, the treatment with the alfa-2 antagonist is preferably started at the same time as the treatment with the dopaminergic agent. The precise amount of the drug to be administered to a mammal according to the present invention is dependent on numerous factors known to one skilled in the art, such as, the compound to be administered, the general condition of the patient, the condition to be treated, the desired duration of use, the type of mammal, the method and route of administration etc. For example, for atipamezole given together with L-dopa, the usual daily dosage will be from 1 to 50 mg, preferably from 10 to 30 mg, divided in 1 to 4 individual doses. Thus, the most preferable single dose for atipamezole will be 10 mg. The alfa-2 antagonist is preferably given simultaneously with the dopaminergic agent.

Typical routes of administration include, without limitation, oral, transdermal, transmucosal, and parenteral routes.

The invention will be further clarified by the following example, which is intended to be purely exemplary of the invention.

### EXAMPLE 1

The effects of atipamezole on the locomotor hyperactivity induced by repeated administration of D-amphetamine were studied in male mice.

### Animals

Experiments were performed with C57BL/6J strain male mice from Jackson Laboratories. Mice were transferred to laboratory at least 2 weeks prior to use. The mice were from 8 to 20 weeks of age at the beginning of an experiment. Groups of 10 mice were housed in standard polypropylene cages (38 X 22 X 15 cm) with free access to standard certified pelleted food (RM1 Maintenance Expanded SQC; Special Diet Services, Essex, UK) and water. Ambient temperature was 22 ± 1 C°, and a 12:12 h light/dark cycle was maintained with lights on at 6 A.M. All experiments were carried out between 7 A.M. and 5 P.M. The animal care was performed in accordance with International Council for Laboratory Animal Science (ICLAS) guidelines.

### Drugs

D-Amphetamine sulphate (Sigma, St. Louis, MO, U.S.A.) and atipamezole HCl (Orion Corporation, Orion Pharma, Turku, Finland) were dissolved in saline (0.9% NaCl) and administered subcutaneously (s.c.) in a 5 ml/kg volume.

### Motor Activity Testing

The locomotor activity of the mice was measured in transparent standard polypropylene animal cages (38 X 22 X 15 cm) with transparent cover and aspen bedding on the floor. Test cages were placed middle of the photobeam frame system (Photobeam Activity System PAS, Cage Rack, San Diego Instruments, San Diego, CA). Computer control unit registered the interruptions of photobeams from 16 individual cages. Three different types of movements were monitored: 1) ambulations (large horizontal movements), 2) fine movements (smaller horizontal movements) and 3) rearings (vertical movements). Locomotor activity was measured at 5-min intervals for 2 h immediately after D-amphetamine or saline administrations.

### Sensitization schedule and atipamezole treatment

D-amphetamine was administered subcutaneously (s.c.) at the dose of 2 mg/kg. Atipamezole was administered s.c. at the dose of 1 mg/kg 20 min before locomotor activity measurement.

In the chronic treatment group mice were administrated during eight days to elicit provoked locomotor hyperactivity to D-amphetamine and the effect of the atipamezole to the locomotor activity. Mice groups in the chronic treatment schedule were saline, saline-amphetamine, amphetamine, atipamezole and atipamezole-amphetamine. A day before experiment, mice were habituated to the test environment. Test groups with different drug treatments were administrated during four consecutive days. At days five and six there were no drug administrations and motor activity testing. At days seven and eight, the produced locomotor hyperactivity and effect of a single exposure of D-amphetamine (saline-amphetamine -group) were analysed. (Table 1).

**Table 1**

| Chronic treatment | | | | | |
|---|---|---|---|---|---|
| Time | saline | saline- amphetamine | amphetamine | atipamezole | atipamezole-amphetamine |
| Habituation | saline | saline | saline. | saline | saline |
| Day 1 | saline | saline | saline | saline | atipam. and amph. |
| Day 2 | saline | saline | amph. | atipam. | atipam. and amph. |
| Day 3 | saline | saline | amph. | atipam. | atipam. and amph. |
| Day 4 | saline | saline | amph. | atipam. | atipam. and amph. |
| Day 5 | no injection | no injection | no injection | no injection | no injection |
| Day 6 | no injection | no injection | no injection | no injection | no injection |
| Day 7 | saline | saline | amph. | atipam. | atipam. and amph. |
| Day 8 | saline | amph. | amph. | atipam. | atipam. and amph. |

At day nine, the effect of different atipamezole and amphetamine administrations to the locomotor activity on the chronic treatment groups were analysed. Used treatments were saline, 1 mg/kg atipamezole, 2 mg/kg D-amphetamine, 0,3 mg/kg atipamezole- 2 mg/kg D-amphetamine and 1 mg/kg atipamezole- 2 mg/kg D-amphetamine. Chronic treatment groups were saline-, atipamezole-, amphetamine- and atipamezole-amphetamine groups. Chronic groups were treated following schedule in Table 2.

**Table 2**

| Drug treatments at day 9 | | | | |
|---|---|---|---|---|
| | Chronic group | | | |
| Drug treatment | saline | atipamezole | amphetamine | atipamezole-amphetamine |
| saline | Yes | No | No | No |
| 1 mg/kg atipamezole | Yes | Yes | No | No |
| 2 mg/kg amphetamine | Yes | Yes | Yes | Yes |
| 0,3 mg/kg atipamezole- 2 mg/kg amphetamine | Yes | No | Yes | No |
| 1 mg/kg atipamezole- 2 mg/kg amphetamine | Yes | No | Yes | Yes |

All data are presented as mean ± SEM. Statistical analysis were performed using SPSS 9.0 for Windows (SPSS, Chicago, IL). Separate repeated measures analyses of variance (ANOVA) were performed on each variable for each experiment grouped on time and treatment group. Results were analysed separately, because data were collected in separate experiments with different study design. When significance (P<0.05) between treatment groups were found comparisons at each time point (date or min) were analyzed by using LSD post-hoc test.

### RESULTS

### Locomotor activity

### Effect of repeated administration of D-amphetamine and atipamezole in chronic treatment groups

Figure 1 illustrates the development of behavioural sensitization after six repeated administrations of D-amphetamine (2 mg/kg) and the effect of atipamezole (1 mg/kg) pre-treatment 20 minutes before D-amphetamine challenge in mice. There was a significant difference between the chronic treatment groups [F(1,184)=1618.9, P<0.001]. The activity counts were dependent on the administration Day [F(6,1104) =107.7, P<0.001 and there was a significant interaction between Day X Group [F(24,1104) = 53.2, P<0.001]. Mice treated with D-amphetamine of six consecutive days (group amphetamine-amphetamine) showed a progressive enhance in ambulatory activity compared to saline group. At Day eight, mice from group saline-amphetamine were also administered with D-amphetamine, but there was still a significant difference compared group amphetamine to groups saline and saline-amphetamine(P<0.001).

Atipamezole pre-treatment before saline challenge tended to enhance locomotor activity after daily atipamezole for six days, but there was not statistical significant difference between the groups atipamezole-saline and saline at Day eight (P=0.43).

Atipamezole pre-treatment before D-amphetamine administration over the course of exposure decreased significantly D-amphetamine-induced locomotion in mice. There was statistical significant difference between groups amphetamine-amphetamine and atipamezole-amphetamine at Day 2 (P<0.05) and Days 3-8 (P<0.001). This indicates that atipamezole pre-treatment prevented D-amphetamine-induced locomotor hyperactivity over the course of the exposure.

### Effect of the different drug treatments at Day 9

Figure 2 illustrates the effect of the different drug treatments in chronic treatment groups at Day 9. Atipamezole pre-treatment alone did not alter total ambulatory activity counts in the saline group (P=0.81). D-amphetamine significantly enhanced locomotor activity in the saline group (P<0.001), but the activity was not same level compared to amphetamine group (P<0.001). Atipamezole (0.3 mg/kg) pre-treatment 20 minutes before D-amphetamine challenge amplified locomotor activity (P=0.016), but with dosage 1 mg/kg had no effect compared D-amphetamine alone (P=0.364). (Figure 2, Group saline).

Atipamezole (1 mg/kg) pre-treatment over the course of exposure had tendency to elevate locomotor activity before saline and D-amphetamine challenge, but elevation was not statistically significant (Figure 2, Group atipam.).

Mice in Group amphetamine developed strong ambulatory sensitization during repeated administration of D-amphetamine (2 mg/kg) (P<0.001, Figure 2). Atipamezole doses (0.3 and 1 mg/kg) both clearly attenuated the D-amphetamine-induced locomotor hyperactivity (0.3 mg/kg P=0.005; 1 mg/kg P<0.001).

Atipamezole pre-treatment (1 mg/kg) 20 min before D-amphetamine administration over the course of exposure (Group atipamezole-amphetamine) decreased significantly D-amphetamine induced locomotion. There was statistical significant difference between groups amphetamine-amphetamine and atipamezole-amphetamine also at Day 9 (P<0.001). Part of mice from Group atipamezole-amphetamine were administrated only with D-amphetamine (2 mg/kg) at Day 9 and there was still statistical significant difference compared to amphetamine-amphetamine -group (P=0.026). This latter result is important as it shows that atipamezole causes a true reduction in the development of sensitization not just the suppression of this expression.

## Claims

1. A use of an alfa2-adrenoceptor antagonist in the manufacture of a medicament for the prevention of the development of sensitization caused by chronic use of dopaminergic agents.

2. The use according to claim 1 wherein the sensitization is dyskinesia seen in Parkinson's Disease after chronic treatment of L-dopa.

3. The use according to any one of claims 1-2, wherein the alfa2-adrenoceptor antagonist is atipamezole or a pharmaceutically acceptable salt thereof.

4. The use according to any one of claims 1-2, wherein the alfa2-adrenoceptor antagonist is idazoxan or a pharmaceutically acceptable salt thereof.

5. The use according to any one of claims 1-2, wherein the alfa2-adrenoceptor antagonist is efaroxan or a pharmaceutically acceptable salt thereof.

6. The use according to any one of claims 1-2, wherein the alfa2-adrenoceptor antagonist is 4-(2-ethyl-5-fluoro-2,3-dihydro-1H-inden-2-yl)-1H-imidazole or a pharmaceutically acceptable salt thereof.

## Patentansprüche

1. Verwendung eines alfa2-Adrenozeptor-Antagonisten bei der Herstellung eines Medikaments zur Verhinderung der Entwicklung der durch chronische Anwendung von dopaminergischen Mitteln verursachten Sensibilisierung.

2. Verwendung nach Anspruch 1, wobei die Sensibilisierung Dyskinisie ist, die bei Parkinson-Krankheit nach chronischer Behandlung von L-Dopa beobachtet wird.

3. Verwendung nach einem der Ansprüche 1 bis 2, wobei der alfa2-Adrenozeptor-Antagonist Atipamezol oder eines seiner pharmazeutisch annehmbaren Salze ist.

4. Verwendung nach einem der Ansprüche 1 bis 2, wobei der alfa2-Adrenozeptor-Antagonist Idazoxan oder eines seiner pharmazeutisch annehmbaren Salze ist.

5. Verwendung nach einem der Ansprüche 1 bis 2, wobei der alfa2-Adrenozeptor-Antagonist Efaroxan oder eines seiner pharmazeutisch annehmbaren Salze ist.

6. Verwendung nach einem der Ansprüche 1 bis 2, wobei der alfa2-Adrenozeptor-Antagonist 4-(2-Ethyl-5-fluoro-2,3-dihydro-1 H-inden-2-yl)-1H-imidazol oder eines seiner pharmazeutisch annehmbaren Salze ist.

## Revendications

1. Utilisation d'un antagoniste du récepteur adrénergique alpha 2 dans la fabrication d'un médicament destiné à la prévention du développement d'une sensibilisation provoquée par une utilisation chronique d'agents dopaminergiques.

2. Utilisation selon la revendication 1, dans laquelle la sensibilisation est la dyskinésie observée dans la maladie de Parkinson après un traitement chronique à la L-dopa.

3. Utilisation selon l'une quelconque des revendications 1 à 2, dans laquelle l'antagoniste du récepteur adrénergique alpha 2 est l'atipamézole ou un sel pharmaceutiquement acceptable de celui-ci.

4. Utilisation selon l'une quelconque des revendications 1 à 2, dans laquelle l'antagoniste du récepteur adrénergique alpha 2 est l'idazoxan ou un sel pharmaceutiquement acceptable de celui-ci.

5. Utilisation selon l'une quelconque des revendications 1 à 2, dans laquelle l'antagoniste du récepteur adrénergique alpha 2 est l'efaroxan ou un sel pharmaceutiquement acceptable de celui-ci.

6. Utilisation selon l'une quelconque des revendications 1 à 2, dans laquelle l'antagoniste du récepteur adrénergique alpha 2 est le 4-(2-éthyl-5-fluoro-2,3-dihydro-1H-indén-2-yl)-1H-imidazole ou un sel pharmaceutiquement acceptable de celui-ci.
